# EUROPEAN PATENT APPLICATION

(11) **EP 4 212 088 A1**
(43) Date of publication of application: **19.07.2023**
(21) Application number: 23150873.0
(22) Date of filing: 10.01.2023
(51) Int. Cl.: A61B 1/00, A61M 25/00

(54) **ERGONOMIC CATHETER HANDLE**

(30) Priority: 12.01.2022 US 202263298830 P; 19.12.2022 US 202218068210
(71) Applicant: Canon U.S.A., Inc., Melville, New York 11747 (US)
(72) Inventor: ALTSHULER, Alexander, Irvine, 92618 (US)
(74) Representative: TBK

(57) **Abstract**

A handle for maneuvering a catheter, comprises: a handle body having an outer surface and an inner surface, the inner surface defining a tubular passage extending along a longitudinal axis from a proximal end to a distal end of the handle body. The handle includes a coupling mechanism at the proximal end and a strain relief sleeve at the distal end of the handle body. The strain relief sleeve is adapted to irremovably secure a flexible catheter sheath to the handle; and the coupling mechanism is adapted to detachably connect the handle to a patient interface unit (PIU). The handle body is ergonomically sized and shaped for grasping by a single hand of a user, and is configured to linearly engage to and disengage from the PIU via the coupling mechanism. When the handle is attached to the PIU, an imaging core moves linearly inside the tubular passage without bending.

## Description

### CROSS-REFERENCE TO RELATED APPLICATIONS

This application is related to, and claims priority from, co-pending U.S. Provisional Application Ser. No. 63/298830, filed 01/12/2022, the content of which is herein incorporated by reference in its entirety.

### BACKGROUND INFORMATION

### Field of Disclosure

The present disclosure generally relates to medical devices. More particularly, the present disclosure is directed to one or more embodiments of a handle connectable to a catheter probe or endoscope, and addresses sterility concerns and device vulnerability when interfacing the catheter or endoscope with a patient interface unit during an interventional procedure.

### Description of Related Art

Using a catheter or endoscope for observing and manipulating organs inside a patient's body is a widely accepted practice in the medical field. In the current state of the art, there is a variety of catheter systems configured for observing and/or maneuvering inside bodily lumens, such as the vascular system, the respiratory system or the digestive system, of a patient's body. An example of such systems is a catheter-based optical coherence tomography (OCT) system, which generally includes a catheter probe containing an optical imaging component (an imaging core) that scans the inside of a bodily lumen. Due to sterility requirements and to minimize the catheter length, catheters are frequently connected to a small intermediate device often called a Patient Interface Unit (PIU), which is placed directly next to a patient, rather than in the main system console. While a catheter would normally be a sterile single-use tool for entering the patient's body, the PIU is reusable and non-sterile.

In a typical procedure, the PIU is draped with a sterile barrier, a catheter is then connected to the PIU, and the assembled device is placed in an operating table or patient bed directly next to a patient to minimize the overall length of the catheter. The attachment of the catheter to the PIU is a complicated procedure that requires good coordination between the sterile and unsterile components. For example, the procedure of attaching the catheter to the PIU requires good coordination between a physician maneuvering the sterile components and an assistant maneuvering unsterile components to prevent contamination of the sterile field and/or sterile components. Therefore, catheter connectors have an important function of securely connecting the catheter to the PIU, and keeping the catheter probe aligned with the pullback system, while preventing contamination between sterile and unsterile components. At the same time, the placement of draped unsterile electronic components next to the patient reduces the space available for the interventions and limits the degrees of freedom for surgeons to operate on the patient. Specifically, in reduced spaces, surgeons have to adopt awkward postures for handling and maneuvering the catheter device and surrounding components. This situation can cause physical fatigue and musculoskeletal disorders especially in the arms, wrists and hands of surgeons. Therefore, the ergonomic characteristics of catheter handles is an important aspect that can alleviate surgeons fatigue, reduce the space taken up by the catheter system, and improve interventional outcomes which is advantageous to the patient.

Some known catheter connectors include those described in patent literature including U.S. Patents Nos.: 5364352, 6783521, 9533122, 10874287, and pre-grant patent application publications including US 2008/0275389, US 2018/0168675, and US 2019/0274666. Other examples of catheter connectors include commercially available systems including TVC Nexus^{™} Controller and TVC Insight^{™} Catheter from Infraredx, Inc., Dragonfly^{™} Imaging catheter from Saint Jude Medical, Inc. (Dragonfly is a trademark of Lightlab Imaging Inc.), Eagle Eye^{™} Gold Intravascular Ultrasound Imaging catheter available from Volcano Corporation, and Terumo hybrid IVUS-OCT catheter system catheter connector. The above listed patent publications are incorporated by reference herein for all purposes. These known catheter connectors are either configured for handheld (manual) operation, or generally focus on connecting the catheter probe to the PIU unit without addressing the issues of sterility, ergonomics and/or user comfort. However, handling a PIU during attachment to the catheter probe, especially during draping and undraping, and maintaining the necessary sterility during preparation and procedural operations, remains a significant concern that could negatively affect the outcome of an intervention. In addition, it has been widely acknowledged that a user's hand size is a significant determinant of difficulty in using surgical instruments and affects the control of catheter and endoscope instruments. See, for example, Ramon Sancibrian et al., "Design and evaluation of a new ergonomic handle for instruments in minimally invasive surgery", Journal of Surgical Research 188 (2014) pages 88-99, Filisetti et al., "Analysis of Hand Size and Ergonomics of Instruments in Pediatric Minimally Invasive Surgery", Surg Laparosc Endosc Percutan Tech. 2015 Oct;25(5):e159-62, and Matsuzaki et al., "Effects of endoscopy-related procedure time on musculoskeletal disorders in Japanese endoscopists: a cross-sectional study", Endosc Int Open: E674-E683, 2021.

The above background is provided to illustrate exemplary technology areas where some embodiments of a catheter handle described in the present application may be practiced to improve the existing technology.

### SUMMARY OF EXEMPLARY EMBODIMENTS

According to at least one embodiment of the present disclosure, a novel catheter probe design has an ergonomic handle that provides improved handling of a PIU device during intraluminal medical operations. The novel catheter probe is an intravascular catheter having an ergonomic handle sized and shaped for grasping by either hand of a user. In one embodiment, a handle for maneuvering a catheter probe inside body lumina is disclosed. A medical apparatus comprises a PIU and a catheter probe removably attachable to the PIU. The catheter probe comprises at least an elongated single part handle and a catheter probe. The elongated handle includes a reinforced proximal section and a strain relief distal section. The catheter probe includes at least one working channel spanning from a proximal end to a distal end of the catheter probe. In one embodiment, the working channel is configured to pass therethough an imaging core having a connector at a proximal end of the catheter probe and a distal optics assembly at the distal end of the catheter probe. The elongated handle is engaged linearly to the patient interface unit such that a pullback unit moves the imaging core a predetermined pullback length in a linear direction. A shape and size of the handle is ergonomically designed to enable a user single hand operation. A length of the elongated handle is determined taking into consideration the predetermined pullback length of the imaging core. In one embodiment, the length of the handle is equal to or greater than the pullback length of the imaging core.

According to another embodiment, a medical system comprises: a system console in direct communication with a patient interface unit, and a flexible catheter probe having an elongated catheter handle. The catheter handle is ergonomically sized and shaped for grasping by a hand of a user for easy attachment of the catheter probe to the patient interface unit. The catheter handle is adapted for support, handling, and manipulations of the patient interface unit without bending or damaging the catheter probe. In an embodiment, the flexible catheter probe includes a flexible catheter sheath and an imaging core adapted for linear pullback motion along a working channel of the catheter probe. At least a proximal section of the catheter probe is supported and protected by a reinforced proximal section and a strain relief distal section of the catheter handle.

Advantageously, the ergonomically sized and shaped catheter handle provides (1) improved ease of handling of the PIU, especially during engagement and disengagement between handle and PIU, during draping/undraping and handouts from non-sterile to sterile fields and vice versa, and (2) complementary rigid heavy duty protection from bending of the probe's imaging core at the proximal portion of the catheter probe.

These and other objects, features, and advantages of the present disclosure will become apparent to those skilled in the art upon reading the following detailed description of exemplary embodiments in conjunction with the appended drawings and provided claims.

### BRIEF DESCRIPTION OF DRAWINGS

Throughout the figures, the same reference numerals and characters, unless otherwise stated, are used to denote like features, elements, components or portions of the illustrated embodiments.
FIG. 1 is an exemplary diagram of a catheter-based medical imaging system 100;
FIG. 2 illustrates an exemplary patient interface unit (PIU) 10 attached to a catheter 20, according to the present disclosure;
FIG. 3 illustrates the exemplary catheter 20 disengaged from the PIU, showing a process of attaching or detaching the catheter 20 to or from the PIU 10, according to the present disclosure;
FIG. 4 illustrates a cross-sectional view of a catheter handle 24 attached to the PIU 10 showing a "Detail A" of tubular passage 51 provided inside the catheter handle 24;
FIG. 5A illustrates a cross-sectional rendering in the lengthwise direction of the catheter handle 24, and FIG. 5B illustrates an axial cross-sectional view of the catheter handle 24, according to an embodiment of the present disclosure; and

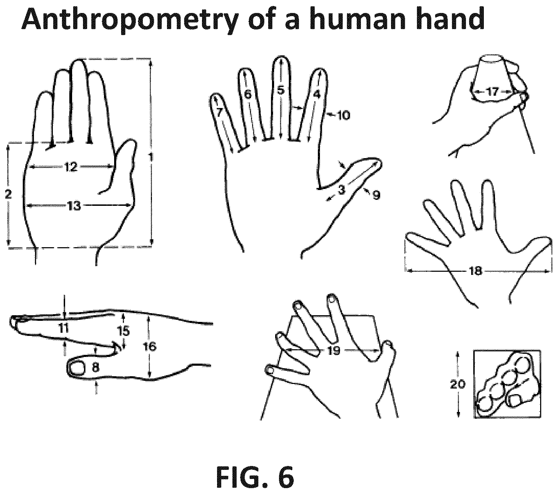

FIG. 6 illustrates anthropometric dimensions of a human hand, as given in Table 1.

### DETAILED DESCRIPTION OF EXEMPLARY EMBODIMENTS

Before the various embodiments are described in further detail, it is to be understood that the present disclosure is not limited to any particular embodiment. It is also to be understood that the terminology used herein is for the purpose of describing exemplary embodiments only, and is not intended to be limiting. More specifically, while the subject disclosure is described in detail with reference to the enclosed figures, it is done so in connection with illustrative exemplary embodiments. It is intended that changes and modifications can be made to the described exemplary embodiments without departing from the true scope of the subject disclosure as defined by the appended claims. Although the drawings represent some possible configurations and approaches, the drawings are not necessarily to scale and certain features may be exaggerated, removed, combined or partially sectioned to better illustrate and explain certain aspects of the present disclosure. The descriptions set forth herein are not intended to be exhaustive, or otherwise limit or restrict the claims to the precise forms and configurations shown in the drawings and disclosed in the following detailed description.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached", "coupled" or the like to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown in one embodiment can apply to other embodiments. It will also be appreciated by those of skill in the art that references to a structure or feature that is disposed "adjacent" to another feature may have portions that overlap or underlie the adjacent feature.

The terms first, second, third, etc. may be used herein to describe various elements, components, regions, parts, and/or sections. It should be understood that these elements, components, regions, parts and/or sections are not limited by these terms of designation. These terms of designation have been used only to distinguish one element, component, region, part, or section from another region, part, or section. Thus, a first element, component, region, part, or section discussed below could be termed a second element, component, region, part, or section merely for purposes of distinction but without limitation and without departing from structural or functional meaning.

As used herein, the singular forms "a", "an", and "the" are intended to include the plural forms as well, unless the context clearly indicates otherwise. It should be further understood that the terms "includes" and/or "including", "comprises" and/or "comprising", "consists" and/or "consisting" when used in the present specification and claims, specify the presence of stated features, integers, steps, operations, elements, and/or components, but do not preclude the presence or addition of one or more other features, integers, steps, operations, elements, components, and/or groups thereof not explicitly stated. Further, in the present disclosure, the transitional phrase "consisting of" excludes any element, step, or component not specified in the claim. It is further noted that some claims or some features of a claim may be drafted to exclude any optional element; such claims may use exclusive terminology as "solely," "only" and the like in connection with the recitation of claim elements, or it may use of a "negative" limitation.

The term "about" or "approximately" as used herein means, for example, within 10%, within 5%, or less. In some embodiments, the term "about" may mean within measurement error. In this regard, where described or claimed, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/-0.1% of the stated value (or range of values), +/-1% of the stated value (or range of values), +/-2% of the stated value (or range of values), +/-5% of the stated value (or range of values), +/-10% of the stated value (or range of values), etc. Any numerical range, if recited herein, is intended to be inclusive of end values and includes all sub-ranges subsumed therein, unless specifically stated otherwise. As used herein, the term "substantially" is meant to allow for deviations from the descriptor that do not negatively affect the intended purpose. For example, deviations that are from limitations in measurements, differences within manufacture tolerance, or variations of less than 5% can be considered within the scope of substantially the same. The specified descriptor can be an absolute value (e.g. substantially spherical, substantially perpendicular, substantially concentric, etc.) or a relative term (e.g. substantially similar, substantially the same, etc.).

Unless specifically stated otherwise, as apparent from the following disclosure, it is understood that, throughout the disclosure, discussions using terms such as "processing," "computing," "calculating," "determining," "displaying," or the like, refer to the action and processes of a computer system, or similar electronic computing device, or data processing device that manipulates and transforms data represented as physical (electronic) quantities within the computer system's registers and memories into other data similarly represented as physical quantities within the computer system memories or registers or other such information storage, transmission or display devices. Computer or electronic operations described in the specification or recited in the appended claims may generally be performed in any order, unless context dictates otherwise. In addition, although various operational flow diagrams are presented in a sequence(s), it should be understood that the various operations may be performed in other orders than those which are illustrated or claimed, or operations may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "in response to", "related to," "based on", or other like past-tense adjectives are generally not intended to exclude such variants, unless context dictates otherwise.

The present disclosure generally relates to medical devices, and it exemplifies embodiments of a catheter probe which may be applicable to a spectroscopic apparatus (e.g., an endoscope), an optical coherence tomographic (OCT) catheter, an intravascular ultrasound (IVUS) catheter, or a combination of such apparatuses. The embodiments of a probe and portions thereof are described in terms of their state in a three-dimensional space. As used herein, the term "position" refers to the location of an object or a portion of an object in a three-dimensional space (e.g., three degrees of translational freedom along Cartesian X, Y, Z coordinates); the term "orientation" refers to the rotational placement of an object or a portion of an object (three degrees of rotational freedom--e.g., roll, pitch, and yaw); the term "posture" refers to the position of an object or a portion of an object in at least one degree of translational freedom and to the orientation of that object or portion of object in at least one degree of rotational freedom (up to six total degrees of freedom); the term "shape" refers to a set of posture, positions, and/or orientations measured along the elongated body of the object.

As it is known in the field of medical devices, the terms "proximal" and "distal" are used with reference to the manipulation of an end of an instrument extending from the user to a surgical or diagnostic site. In this regard, the term "proximal" refers to the portion (e.g., a handle) of the instrument closer to the user, and the term "distal" refers to the portion (tip) of the instrument further away from the user and closer to a surgical or diagnostic site. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

As used herein the term "catheter" generally refers to a flexible and thin tubular instrument made of medical grade material designed to be inserted through a narrow opening into a bodily lumen (e.g., a vessel) to perform a broad range of medical functions. The more specific term "optical catheter" refers to a medical instrument comprising an elongated bundle of one or more flexible light conducting fibers disposed inside a protective sheath made of medical grade material and having an optical imaging function. A particular example of an optical catheter is fiber optic catheter which comprises a sheath, a coil, a protector, and an optical probe. In some applications a catheter may include a "guide catheter" which functions similarly to a sheath.

In the present disclosure, the terms "optical fiber", "fiber optic", or simply "fiber" refers to an elongated, flexible, light conducting conduit capable of conducting light from one end to another end due to the effect known as total internal reflection. The terms "light guiding component" or "waveguide" may also refer to, or may have the functionality of, an optical fiber. The term "fiber" may refer to one or more light conducting fibers. An optical fiber has a generally transparent, homogenous core, through which the light is guided, and the core is surrounded by a homogenous cladding. The refractive index of the core is larger than the refractive index of the cladding. Depending on design choice some fibers can have multiple claddings surrounding the core.

Many medical devices require a sterilized component capable of extending into a sterile field, without risk of contaminating that field, where that sterile component interfaces with an unsterile component needed to operate the device. In this situation, the unsterile component can potentially enter the sterile field and negate the sterility of the sterile field. A solution to prevent loss of sterility has been to deploy a sterile barrier over any unsterile component entering the sterile field. This technique especially applies to medical devices used in *in-vivo* environments, where sterile components such as interventional catheters must interface with an electronic controller which cannot be sterilized.

An example of *in-vivo* deployable catheter system includes an optical coherence tomography (OCT) catheter, which includes a catheter probe containing an optical imaging component (an imaging core) that interfaces with a drive system that enables catheter movement for imaging functionality. The drive system in turn interfaces with a computer system inside a console, responsible for digital operations of the system. Another example includes an intravascular ultrasound (IVUS) catheter system which also includes a catheter probe containing an ultrasound transducer component that interfaces with a pullback system enabling catheter deployment and imaging functionality. These types of catheter systems can sometimes be combined to form multimodality catheter systems such as a multimodality OCT system (MMOCT system) which combines OCT and fluorescence imaging modalities, or a multimodality NIRS-IVUS system which combines IVUS and near infrared spectroscopy modalities.

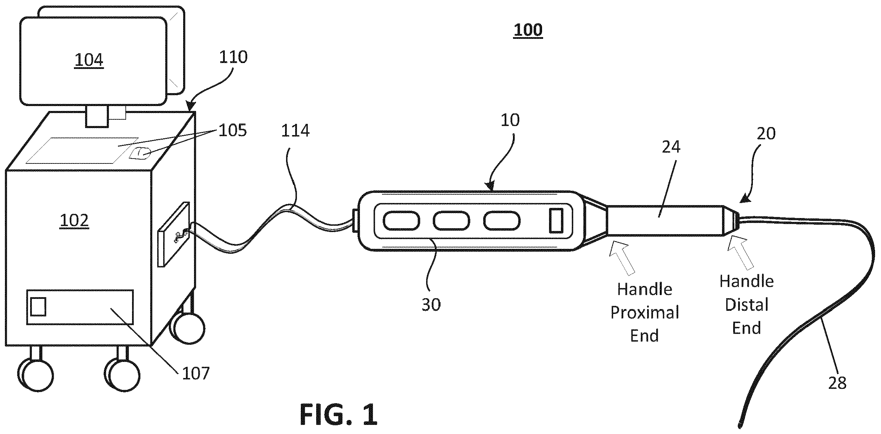

FIG. 1 shows an exemplary medical imaging system 100 which may include any of an OCT, MMOCT, IVUS, NIRS-IVUS, or other similar catheter-based medical imaging system. The medical imaging system 100 includes a system console 110 and a catheter 20 joined to each other via a cable bundle 114 and a patient interface unit (PIU) 10. The system console 110 includes, among other components, a cart 102, one or more monitor displays 104 (e.g. an LCD or OLED display), user interface components 105 (e.g., a keyboard and mouse), and a computer system 107. The PIU 10 is the main interface between the catheter 20 which is typically a single-use sterile component, and the system console 110 which includes non-sterile components.

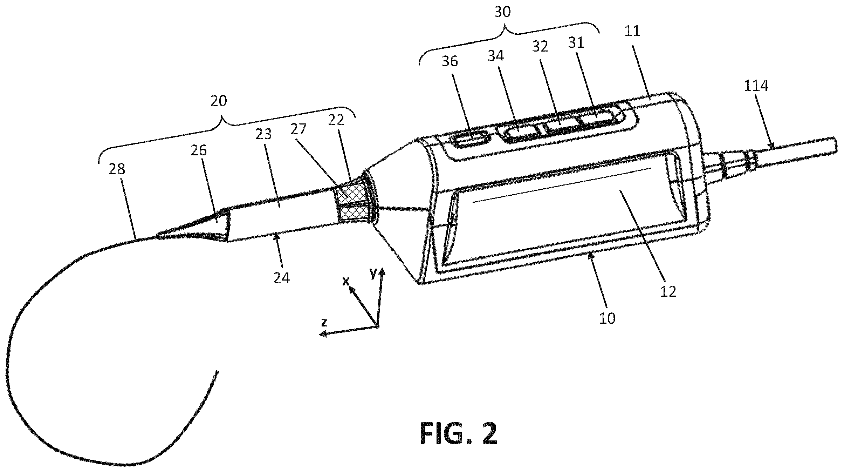

FIG. 2 shows an exemplary illustration of the catheter 20 attached to the PIU 110. The catheter 20 consists of a catheter probe 28 (a probing shaft) and a handle 24. The handle 24 is substantially thicker than the probing shaft, and is configured to be grasped by a single hand of a user. The catheter probe 28 is an elongated, thin and flexible shaft configured to be inserted into a bodily lumen of a patient to probe and examine a target location inside the bodily lumen. In at least some embodiments, catheter probe 28 can be provided with a distal section that can be remotely manipulated, for example, by one or more actuating wires (not shown).

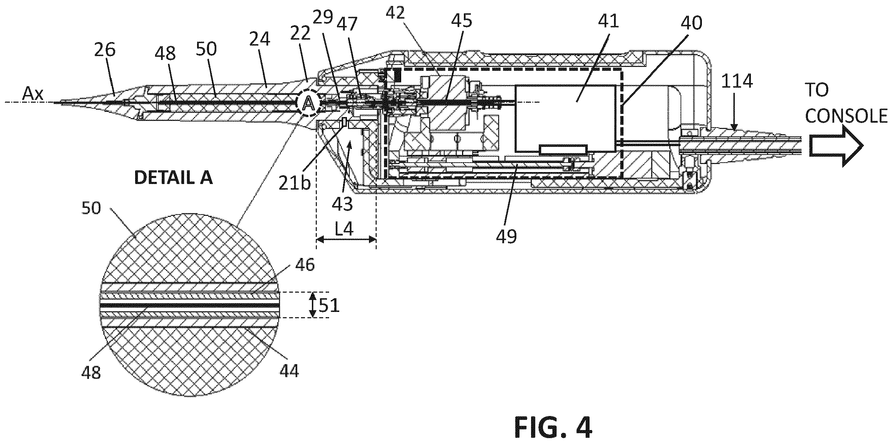

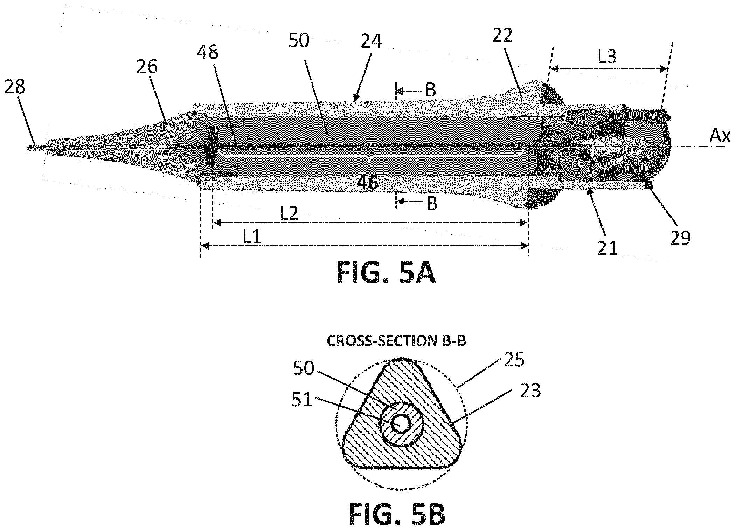

In the catheter 20, a mechanically rotatable imaging core 48 (see FIG. 4) spans through the catheter handle 24 to the distal tip of the catheter probe 28 along a longitudinal axis Ax. The handle 24 has a proximal end and a distal end extending along the longitudinal axis Ax, and provides a tubular passage 51 for the imaging core 48 (see FIG. 5A). In an exemplary imaging procedure, the system console 110 controls movement (rotation and linear displacement) of the imaging core 48 inside the catheter 20 via the PIU 10 to obtain images of a target location (not shown) from inside a bodily lumen, such as a blood vessel in the vasculature of the patient.

The PIU 10 provides the means to rotate and linearly translate the catheter's imaging core 48 (see FIG. 4) within the catheter's stationary outer sheath (not labeled). The system console 110 and PIU 10 are connected to each other by the cable bundle 114. The cable bundle 114 encloses therein electrical cables for transmitting electrical power to the PIU, and cables for signal communication between the PIU 110 and the computer system 107, as well as optical fibers for delivery and collection of light. Depending on the modality of the medical imaging system 100, the signals transmitted through the cable bundle 114 may include one or more of electrical, optical, or other types of electromagnetic radiation.

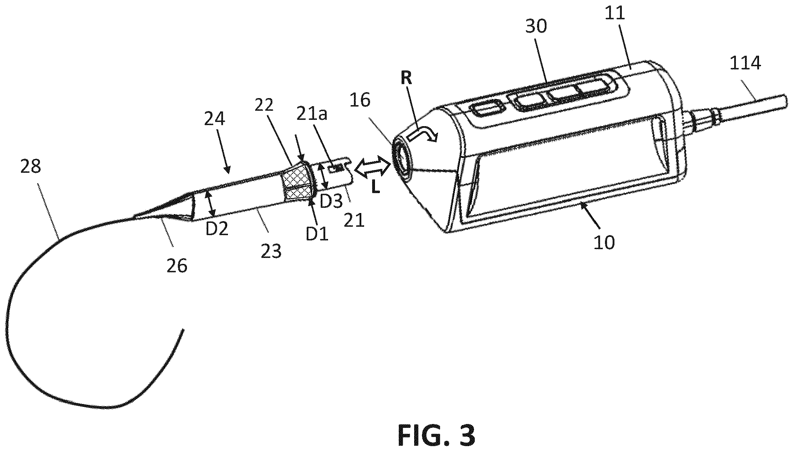

Before use, the PIU 10 is normally stored within the system console 110 without the catheter 20 being attached. The shape of a PIU housing 11 is optimized to facilitate easy gripping and handling for rapid deployment. During use of medical imaging system 100, the entire PIU 10 is removed from the console, covered with a sterile drape (not shown), and placed on the patient's bed or operating table near the patient. Alternatively, the PIU is covered by the sterile drape during (at the time of) attachment of the catheter 20. Once the PIU 10 is placed next to the patient, a sterile user (e.g., a surgeon) attaches the catheter 20 to the PIU 10, by carefully aligning the catheter handle 24 and engaging a coupling mechanism 21 to a connection port 16 (receiving port) in a push and twist motion (see FIG. 3). To prevent loss of sterility and facilitate safe attachment of the catheter 20 to the PIU 10, a non-sterile user (e.g., a physician assistant) may hold and/or maneuver the PIU 10 while the sterile user maneuvers the catheter handle 24 to attach the catheter 20 to the PIU 10. After attachment, the handle 24 is configured to support manipulation of the entire PIU 10, by the sterile user grasping the handle 24 and maneuvering the combined assembly single handedly.

The PIU 10 may provide a user interface 30 for operating the imaging functions of the catheter 20 from a sterile field by the use of actionable hardware buttons and/or touchscreen buttons provided on the outer surface of the PIU housing. The buttons or touchscreen of the user interface 30, on the PIU 10, may mirror control buttons provided on a graphical user interface (GUI) at the display 104 of system console 110. The state of each button (e.g., active, inactive) is communicated to the user by indicator light emitting diodes (LEDs), which is also mirrored on the GUI. In this manner, users may perform the same operations using buttons on the GUI or buttons located in the user interface 30.

The PIU 10 comprises a housing 11, a gripping portion 12, and a user interface 30. The distal end of the PIU 10 connects to the catheter 20 via the catheter handle 24, and the proximal end connects to the system console 110 via the cable bundle 114. The gripping portion 12, in one embodiment, is an indentation formed in the lengthwise direction on one or more sides of the housing 11. The gripping portion 12 allows a user to lift and maneuver the PIU housing 11 without accidentally touching other sensitive surfaces or components such as the user interface 30. The user interface 30 may include a plurality of control buttons including a first button 31, a second button 32, and a third button 34, and one or more status indicators, such as an LED panel 36.

The PIU housing 11 can have any shape or form factor as long as the components enclosed therein are securely protected, and the footprint of the device is optimized for ease of maneuvering and handling in sterile environments, such as clinical settings or operating rooms. In one embodiment shown in FIG. 2 and FIG. 3, the PIU hosing 11 has a generally elongated three-dimensional (3D) polygonal shape having opposite proximal and distal ends defined by two-dimensional polygons, and an elongated body defined by side surfaces joining the proximal and distal ends. In some embodiments, the housing 11 may have the proximal and distal ends slightly slanted towards each other such that, when viewed from a side perpendicular to the lengthwise direction, the general shape of the housing is that of an elongated truncated pyramid. In any embodiment, the housing 11 may have a generally rectangular base and one or more (preferably two) slanted side surfaces so that the center of gravity of the housing is at or near the base surface of the housing. This is advantageous for maintaining stability of the PIU unit around other surgical tools.

The PIU housing 11 can be made of well-known materials, such as plastic, resin or metal, and combinations thereof. Depending of the specific application and use, the housing can be made of multiple components, which can be assembled to constitute a three-dimensional shell covering the internal components. In some embodiments, the housing 11 can be injection molded or 3D printed from well-known plastic or polymer resin materials, or from specialized anti-bacterial and/or disinfectant-ready plastic. Alternatively, the housing 11 can be made of sheet metal, cut and bent to multiple sections of a shell to create the three-dimensional shape of the PIU unit. The edges, vertices, and any other sharp edges are chamfered or rounded.

The catheter handle 24 includes a handle body 23 having an outer surface and an inner surface, the inner surface defining a tubular passage with a length extending along a longitudinal axis Ax from a proximal end to a distal end of the handle body. The outer surface of the catheter body can be made of typical medical-grade (i.e., sterilizable) rigid plastic material. The inner surface can be defined by a tube made of dampening material. The handle body 23 includes a coupling mechanism 21 at the proximal end, a strain relief sleeve 26 at the distal end, and a reinforced portion 22 between the proximal end and the distal end. The catheter probe 28 has a proximal portion which is enclosed and fixedly held by the catheter handle 24. The catheter probe 28 exits from the distal end of handle 24 via the strain relief sleeve 26. The strain relief sleeve 26 is disposed immediately adjacent to and including a longitudinal sleeve lumen in mechanical communication with the tubular passage 51. The strain relief sleeve 26, at the distal end of the handle body 23, is adapted to irremovably secure the handle body 23 to the catheter probe 28 which is flexible and thin tubular sheath. The strain relief sleeve 26 includes material having a durometer that allows lateral flexibility, but resists kinking or crimping of the catheter probe 28 extending longitudinally through a longitudinal lumen of the strain relief sleeve.

The handle 24 is adapted in size and shape to provide for an ergonomic grip by a single hand of the user. The catheter handle 24 may have different shapes and sizes. In at least one embodiment, the handle body 23 can have a handle length (L1) substantially equal to or longer than a length (L2) of a pullback portion 46, as shown in FIG. 5A. The pullback portion 46 is the portion of the imaging core 48 that is pulled through the tubular passage 51 and into PIU 10 during a pullback procedure. In order to protect the pullback portion 46 of the imaging core from a bending moment, the length of pullback (the pullback length) is shorter than the length L2 of the pullback portion 46. In terms of shape, the handle body 23 can have a generally triangular cross section fitting into a circle 25, as shown in FIG. 5B. In one example, the handle body 23 has a length L1 of 133 millimeters (mm), and has a generally triangular cross section fitting into a circle 25 of 25 mm diameter. In other embodiments, the handle body 23 has a generally circular cross section having a 25 mm diameter and a 133 mm length. The coupling mechanism 21 has a length L3, which in one embodiment is about 45 mm. Accordingly, the length L3 of the coupling mechanism 21 is about 1/3 of the length L1 of handle body 23 (i.e., L3 ≈(1/3)L1).

The handle body 23 of either embodiment can be provided with slippage prevention knurl-like features 27 on at least part of the outside surface thereof to facilitate a secure grip by the user. In FIG. 2 and FIG. 3, the reinforced portion 22 at the proximal end of the handle is provided with knurl-like features 27 to provide a more secure grip to the user. The length and cross section of the handle body 23 are defined such that the handle 23 avoids transfer of bending moment or vibrations or strain to the imaging core 48 when the catheter is held, gripped, or rotated by the user's hand during manipulation of the device (e.g., imaging operations). It is similarly important to maintain sturdy and consistent alignment in the handle-to-PIU assembly to protect the proximal rigid pullback section (pullback portion) of the probe's imaging core, during twist-push and twist-pull motions for attachment and detachment of the handle 24 to/from the PIU 10.

The catheter probe 28 includes, for example, a flexible catheter sheath having at least one working channel (working lumen) spanning through a proximal end and a distal end of the sheath. The at least one working channel of the catheter probe 28 is aligned with and connected to a similar tubular passage 51 provided inside the handle 24 along the longitudinal axis Ax. In this manner, the working channel of the catheter probe 28 and the tubular passage 51 of the handle body are adapted to pass therethrough one or more of the imaging core 48, fluids, other imaging components (e.g., a removable camera chip), or tools according to the modality or modalities of the medical imaging system 100. In an endoscope, the working channel may accommodate either a fiber-optic bundle for transmitting an optical image back to the proximal end of the endoscope assembly or, alternatively, a video camera chip and the necessary electrical conductors for powering the chip and for transmitting digital signals representative of the image back to the system.

In one embodiment of a MMOCT modality, for example, the working channel of the catheter probe can be configured to pass therethrough an imaging core 48 comprised of one or more optical fibers and a distal optics assembly (not shown). In an OCT catheter, the optical fiber and distal optics assembly components are generally torque-cable driven by being enclosed in a drive cable (torque cable or torque coil) that transfers rotation from a hollow shaft motor 42 to the distal end of the catheter probe 28 to rotate the distal optics assembly (not shown). In an embodiment, the imaging core 48 includes a pullback portion 46 arranged inside the handle body 23 along the tubular passage 51; and the distal optics assembly is arranged at the distal end of the catheter probe 28. The distal optics assembly may include a lens (e.g., a ball lens or GRIN lens) and a beam directing component (e.g., a mirror or a prism). In this case, catheter probe 28 can scan an inner wall of the bodily lumen with a light beam transmitted through a side-view window (not shown) arranged at the distal end of the catheter probe. In other embodiments, the imaging core 48 may include an imaging sensor, such as a chip-on-tip (COT) camera arranged at the distal end of the catheter probe 28. In an embodiment of an IVUS modality, for example, the tubular passage 51 in the handle 24 and the catheter probe 28 may be configured to pass therethrough an imaging core 48 comprised of rotatable torque coil and a rotary ultrasound transducer (not shown).

Here, it should be understood by those skilled in the art, that the present disclosure is not limited to any specific examples of the catheter probe or its working channel or the imaging core arranged therein. Instead, those skilled in the art will appreciate that the present disclosure relates more specifically to the functionality and design of the catheter handle 24 configured to improve connection efficiency and maneuvering of the PIU 10, by a single hand of a user. In that regard, any catheter that has an ergonomic handle connectable to a catheter drive unit (herein referred to as PIU 10) can be implemented according to the principles of the present disclosure.

FIG. 3 shows a process for attaching and/or detaching the catheter 20 to PIU 10, according to the present disclosure. For attachment and detachment, the PIU 10 includes an opening or connection port 16 configured to pass therethrough the coupling mechanism 21 of the catheter handle 24. The coupling mechanism 21 is an engagement portion of the handle 24. The handle 24 includes a reinforced portion 22 at a plane where the handle body 23 abuts against the connection port 16 of the PIU 10; the reinforced portion 22 and the coupling mechanism 21 are made robust enough to support the weight of the entire PIU connected to the handle, while protecting the imaging core from bending moment and vibrations. To maintain the catheter 20 securely coupled to the PIU, the coupling mechanism 21 includes a locking feature 21a, which provides a secure connection between the PIU 10 and the catheter handle 24. The coupling mechanism 21 is either rigidly attached to, or integrally built into, the handle body 23. The coupling mechanism 21 can be made of a metallic tube, and rigidly attached (e.g., bonded, fused, or welded) to the polymer material of the handle body 23. Alternatively, the coupling mechanism 21 can be built into the handle body 23 made by injection molding or 3D printing of plastic or polymer material in a process to form a single component. When made of plastic or polymer material, the coupling mechanism 21 can be optionally reinforced by a metallic structure such as coiled or braided metallic wire, or by polymeric fibers. The coupling mechanism 21 can be a short segment of metallic hypotube (short segment of cylindrical hypotube), and the handle body 23 can be a cylindrical tube made of molded plastic, such that the handle body 23 has an outer surface and an inner surface, the inner surface defines a tubular passage 51 extending along the longitudinal axis from a proximal end to a distal end of the handle body.

To attach the handle 24 to the PIU 10, a user grabs the sterile catheter handle 24 and preforms a push and twist procedure (push-twist motion). Specifically, as shown in FIG. 3, the user grasps the handle 24 from the handle body 23 with a single hand, inserts the coupling mechanism 21 into the opening or connection port 16 in a linear movement L, and rotates the handle 24 in a rotational direction R until the locking feature 21a engages with a corresponding locking feature such as a locking pin 21b (shown in FIG. 4) of PIU 10. The coupling mechanism 21 can be implemented by a bayonet-style mechanism which is a well-known style of mechanical connection in medical devices.

When engagement of the catheter handle 24 is complete, the one or more indicators (e.g., one or more LEDs) provided in the user interface 30 can output a signal (visual) indicative of correct engagement. It is also possible to output a signal indicative of incorrect engagement in an event where incorrect engagement has occurred. Alternatively, the signal of engagement is output when the imaging core 48 has engaged properly with the internal connector of the PIU, to indicate that both the mechanical engagement of the handle and optical connection of the imaging core has been completed. To detach the catheter handle 24 from PIU unit 10, the user will perform a twist and pull procedure (twist-pull motion) opposite to the engagement procedure, at an appropriate state of the PIU internal connection.

The complete PIU 10 and catheter 20 after attachment of the catheter handle 24 is illustrated in FIG. 2. After the catheter handle 24 is locked in place to the PIU 10, the entire assembly behaves as a unitary component (a unibody), which may be easily handled and maneuvered by the user holding the catheter handle 24 with a single hand. The unibody can also be maneuvered by the user holding the PIU from the gripping portion 12. Advantageously, maneuvering the unibody by the user holding the catheter handle 24 can prevent the loss of sterility, which can occur if the user holds the PIU from the gripping portion 12 without a sterile cover.

FIG. 4 illustrates a cross-sectional view of catheter handle 24 attached to exemplary PIU 10. DETAIL A shows the imaging core 48 arranged along the inner tubular passage 51 of the catheter body 23. The PIU 10 is comprised of a pullback unit 40 and a handle receptacle 43. The pullback unit 40 includes a linear stage 49, a fiber optic rotary joint (FORJ) having a hollow shaft motor 42, and beam combiner 41. The hollow shaft motor 42 spins or rotates an optical fiber 45. The PIU 10 also includes electronic control boards (not shown), control buttons (31, 32, 34) and an LED panel (36) for the user interface 30 (shown in FIG. 2), among other components. The handle receptacle 43 includes a locking mechanism 21b, which is configured to engage with the locking feature 21a of the coupling mechanism 21 of handle 24 (see FIG. 2). The catheter receptacle 43 and locking mechanism (not shown) are part of a support base plate to which the linear stage 49 is rigidly attached. The non-illustrated locking mechanism of the support base plate engages with locking feature 21a to provide accurate alignment of the PIU 10 to the catheter handle 24. The support base plate is mounted within the PIU housing 11 on a set of vibration isolating mounts to increase PIU stability and durability.

The catheter receptacle 43 receives the proximal portion (coupling mechanism 21) of the catheter handle 24, and locks the coupling mechanism 21 in place with a locking mechanism such as a spring loaded pin (not shown) configured to engage with the locking feature 21a. In other embodiments, the coupling mechanism 21 of the catheter handle 24 can be configured to be locked in the receptacle 43, by a bayonet type locking mechanism (not shown). In a bayonet type locking mechanism, a cylindrical male side part of the handle (e.g., the coupling mechanism 21) having one or more radial pins (not shown) engages with a cylindrical female receptor having one or more matching L-shaped slots. When at least one of the L-shaped slots of the female receptor engages with at least one of the pins of the cylindrical male side part, the handle 24 remains locked to the PIU in a predetermined orientation. In addition, a secondary locking feature configured to prevent accidental catheter handle disconnection during normal operation can be provided by, for example, controlling disengagement of the locking mechanism 21b from the inside of the PIU using coordinated sequential motions of the hollow shaft motor 42 and the linear stage 41. To control disengagement of the catheter, the system console 110 can be programmed to send control signals to the PIU to stop rotation of the hollow shaft motor 42 and move the linear stage 49 away from the catheter handle, so that the first fiber connector 29 disengages from the optical fiber 45. Furthermore, for safety, there can be provided a user accessible emergency catheter-lock deactivation mechanism (e.g., a push button) in the user interface 30 to automatically stop rotation of the hollow shaft motor 42, and disengage the optical fiber 45 from the fiber connector 29. A method of immobilizing an optical fiber connection is described in U.S. Pat. Ser. No.: 17/394,256 filed by the assignee of the present application on 8/04/2021, herein incorporated by reference in its entirety.

The electronic control boards (not shown) contain all necessary electronic components including a microcontroller to control PIU operations and enable communication with the computer system 107. The control panel can be positioned on the top portion of the PIU attached to the inner surface of the PIU housing 11. The user interface 30 contains a plurality of buttons for quick access to major PIU functions without the need for the user to leave the sterile environment. The buttons of the user interface 30 are recessed within the housing 11 to minimize accidental activation during routine PIU handling. The LED indicators are provided on the PIU's control panel to display current device status.

The beam combiner 41 includes a free space optical system composed of a set of collimated optical fibers, mirrors, and filters for light beam splitting and redirection. The optical rotary joint provides optical coupling between the stationary beam combiner and a rotating optical fiber 45 positioned in a bore of the hollow shaft motor 42. The optical fiber 45 is collimated on the beam combiner facing end and connectorized on the catheter connection end. A field-replaceable fiber connector 47 is attached between the optical fiber 45 arranged in the bore of the hollow shaft motor 42 and the optical connector 29 at the proximal end of the imaging core 48. The hollow shaft motor 42 is mounted on a support platform and is precisely aligned and fixed to the beam combiner 41. The entire rotary joint and beam combiner 41 is then mounted on a moving plate of the linear stage 49. This allows for pullback withdrawal and subsequent advancing of the rotating catheter's imaging core 48 during an imaging procedure.

As shown in FIG. 4, when the catheter 20 is connected to the PIU 10 via the catheter handle 24, the handle body 23, the pullback portion 46 of the imaging core 48, and the hollow shaft motor 42 are aligned collinearly along the longitudinal axis Ax. In this manner, the optical fiber in imaging core 48 and the rotating optical fiber 45 in the hollow shaft motor 42 are linearly connected to each other. Therefore, when linear stage 49 moves the pullback unit 40, the imaging core 48 can move in a linear direction during catheter pullback and advancement, while the imaging core remains protected from bending or vibrations.

FIG. 5A illustrates a perspective view of catheter handle 24 sectioned off in the lengthwise direction along the longitudinal axis Ax. FIG. 5B illustrates a cross-sectional view of the catheter handle 24 taken along a plane (B-B) perpendicular to the longitudinal axis Ax. As shown in FIG. 5A, the catheter probe 28 extends distally away from the handle body 23 through the strain relief sleeve 26. The imaging core 48 is arranged inside the catheter probe 28 along the inner passage 51. A proximal end of the imaging core 48 is fixedly attached to a fiber connector 29 (a first fiber connector), which is arranged in an inner diameter of the coupling mechanism 21. The handle body 23 of catheter handle 24 is configured to be grasped by the user's hand so that the user can insert the coupling mechanism 21 into the receiving port 16 of the PIU 10 in a push and twist motion as shown in FIG. 3. The manual insertion of the catheter handle into the PIU does not connect the imaging core 48 to the optics of the PIU. It connects the handle and the catheter probe to PIU, and then the PIU mechanism connects the imaging core with its motor and fiber connection mechanism.

More specifically, the fiber connector 29, as illustrated in the cross section of FIG. 4, is connected to a corresponding fiber connector 47 (second fiber connector) provided at the distal end of an optical fiber 45 rotated by the hollow shaft motor 42. The optical fiber 45 is driven (rotated or oscillated) by the hollow shaft motor 42 (a rotary drive) of the PIU 10. The hollow shaft motor 42 can be a direct current (DC) brushless motor, which rotates the optical fiber 45 and imparts rotational motion on the fiber connector 29. This allows the imaging core 48 to rotate the distal optics assembly at the distal end of the catheter probe 28 allowing for full 360 degree imaging of a bodily lumen. To be able to acquire an image a particular stretch of a patient's bodily lumen the hollow shaft motor 42 is mounted on a support portion connected to the linear stage 49. The linear stage 49 is capable of moving the hollow shaft motor 42 together with the FORJ and the beam combiner 41 in a linear direction along (parallel to) the motor's rotational axis, and pulling the attached proximal portion of the imaging core 48 to the inside of the PIU. The proximal portion of the rotating imaging core 48, referred herein as a pullback portion 46, has to be self-supported when pulled inside the PIU 10, and, therefore, the pullback portion 46 must be rigid enough to avoid bending of the imaging core 48. To provide the necessary rigidity, the pullback portion 46 is made of metallic hypodermic tubing (i.e., a hypotube), and is disposed inside the catheter handle inner tubular passage 51 (bore) within a stationary low friction tube 44. That is, pullback portion 46 is a metallic hypotube arranged inside a low friction tube 44, which facilitates linear displacement of the imaging core 48. The stationary low friction tube 44 may have a lubricous coating, and is supported within the handle body 23 by a hollow tube of dampening material 50.

The imaging core 48 includes a signal-transmitting waveguide, such as one or more than one optical fiber, placed inside of the pullback portion 46 (a hypotube). When the catheter 20 is attached to the PIU 10 and the system initiates an imaging procedure, the imaging core 48 is rotated and linearly pulled back, while the outer sheath of the catheter probe 28 remains stationary. To provide additional rigidity and prevent non-uniform rotational distortion (NURD), the reinforced portion 22 of the catheter handle 24 increases the diameter of the handle body 23 in a section where the distal end of the PIU housing 11 abuts against the proximal end of the catheter handle 24. In other words, the reinforced portion 22 of the catheter handle 24 is a section of the handle body 23 that provides higher flexural rigidity, e.g., by making a portion of the handle body tapered in a direction towards the distal end from a plane where the distal end of the PIU housing 11 abuts against the proximal end of the catheter handle 24. For example, FIG. 3 shows that reinforced portion 22 tapers from a first diameter D1 at a plane where the proximal end of handle body abuts against the PIU housing 11 to a second diameter D2. In this case, reinforced portion 22 is a portion of the handle 24 having its outer diameter D1 tapered down to the diameter D2 of the handle body 23. The diameter D2 of the handle body can be substantially equal to a diameter D3 of the cylindrical coupling mechanism 21. In turn, a diameter of the receiving port 16 of PIU 10 is dimensioned to engage with the outer diameter D3 of the coupling mechanism 21. A length of the reinforced portion 22 can be about 10% to 25% of the length of the handle body 23.

To provide appropriate flexural rigidity, the handle body 23 can be made by an injection molding process using common medical-grade plastic material or well-known polymers. As mentioned elsewhere, at least portions of the handle body can be reinforced with materials of higher durometer value. In this manner, the reinforced portion 22 can be located anywhere between the proximal end and the distal end of the catheter handle, as long as a portion of the handle body provides higher flexural rigidity to support the pullback portion 46 and protect the imaging core 48 from bending.

At the distal end of the handle body 23, the strain relief sleeve 26 is also tapered in the direction towards the distal end of the catheter probe to provide a smooth transition for the imaging core 48, from the linear and rigid catheter handle to the flexible catheter probe 28. The strain relief sleeve 26 can be an elastic polymer tube with its outer diameter tapered down to the diameter of the catheter sheath. The strain relief sleeve 26 can also be made by injection molding of plastic or resin-based polymers having lower durometer than the handle body 23. As shown in "DETAIL A" of FIG. 4, at least a portion of the catheter handle 24 is axially symmetric and surrounds the imaging core 48 with the reinforced portion 22 so that the user can hold the handle 24, and maneuver the PIU 10 single handedly, especially during PIU draping / undraping and handoffs of the catheter.

It will be appreciated from the foregoing description and illustrations of FIG. 1-5 that the hypotube (pullback portion 46) protruding outside of the handle 24 and moving into the PIU housing 11 during a pullback operation may be easily damaged (bent) if the hypotube were left unsupported by the ergonomic features of the handle 24. If the pullback portion 46 is bent, it will lead to increased rotational friction and added vibrations, degrading image quality due to NURD. To alleviate, minimize, and/or suppress rotational friction and vibrations, a robust reinforced portion 22 surrounding the rigid hypotube is provided. In addition, reinforced portion 22 has the additional benefit of protecting the pullback portion 46 from handling damage during movement and/or positioning (or repositioning) of the PIU 10. To provide the described physical protection for the pullback portion 46, the handle body 23 shall a length L1 at least equal or longer than the desired pullback length. That makes the optimal length of the handle body 23 determined by at least two factors, including (a) the ergonomic grip length of the handle body 23, and (b) the pullback length that the pullback unit moves the imaging core, whichever is greater. Certain commercially available OCT systems have a default maximum pullback length of about, e.g., 10 cm (100 mm). In such OCT systems, the system can automatically acquire and display a first image of a lumen with the maximum pullback length. Then, based on requirements of the imaging procedure (e.g., based on the location and/or size of a stent and/or malapposition thereof), a user would select points from within the first image corresponding to a portion of the maximum pullback length. In that case, after the user selects a point, the imaging core will perform an image capture operation with a reduced pullback length. Here, in this example, the second image will have a resolution higher than that of the first image. In some embodiments, the user or the system may input/send commands to the PIU to perform an imaging procedure, in which the commands may specify the pullback length, pullback rate, and/or image frame rate of the imaging procedure. In that case, the pullback length used to determine the optimal length of the catheter handle should be the default maximum pullback length of the system.

In one embodiment of the present disclosure, the required pullback length is 80 mm, and the catheter handle is 133 mm long. Since the length of the pullback portion 46 is securely contained inside the 133 mm long catheter handle 124, any potential bending of the pullback portion is avoided. In other words, the length of the catheter handle 24 can be in a range of about 1 to 1.5 times the pullback length, or at most in a range of about 1 to 2 times the length of the pullback length. In at least one embodiment, the length of the catheter handle 24 can be determined by considering an average width of a human hand, in addition to (or in combination with) the maximum or standard pullback length of the imaging core.

Therefore, according to the present disclosure, the parameters of catheter handle 24 can be determined by at least two factors including, for example, (A) the ergonomic grip length L1 of the catheter handle which relates to an average width of a human hand, and (B) the pullback length that is related to the length L2 of the pullback portion 46 of the imaging core 48 arranged inside the handle body 23. In addition, the diameter D2 of the catheter handle can be determined based on the ergonomic grip diameter of a human hand (e.g., the overall thickness of the catheter handle can be such that the handle fits into a circle equal to or smaller than a maximum grip diameter of a user's hand). In some embodiments, as long as the pullback portion 46 of the imaging core 48 can be safely protected by the handle body 23, the length and thickness of the handle 24 can be based mainly on the ergonomic parameters of an average size of a user's hand.

Data about an average size of a user's hand is readily available from various publications, such as those discussed in the *Background* section of this disclosure. In addition, the reader can refer to, for example, Table 1 below, which is reproduced from table 5.1 in publication entitled "Bodyspace Anthropometry, Ergonomics and the Design of Work", by Stephen Pheasant, Second Edition, published by Taylor & Francis e-Library, USA, 2003. In Table 1, dimension # 12 provides the sizes for the "Hand breadth", and dimension # 17 provides the "Maximum grip diameter", for the hand of women and men. Herein, "hand breath" is defined as the maximum width across the palm of the hand (at the distal ends of the metacarpal bones), and "*maximum grip diameter*" is defined as a circle measured by sliding the hand down a graduated cone until the thumb and middle fingers only just touch each other. Since a user needs to fully grasp the catheter handle with a single hand, the overall diameter of the catheter handle needs to be smaller than the maximum grip diameter.

**Table 1: dimensions of a human hand given in millimeters (mm)**

| | **Men** | | | | **Women** | | | |
|---|---|---|---|---|---|---|---|---|
| **Dimension** | **5th %ile** | **50th %ile** | **95th %ile** | **SD** | **5th %ile** | **50th %ile** | **95th %ile** | **SD** |
| **1. Hand length** | **173** | **189** | **205** | **10** | **159** | **174** | **189** | **9** |
| **2. Palm length** | **98** | **107** | **116** | **6** | **89** | **97** | **105** | **5** |
| **3. Thumb length** | **44** | **51** | **58** | **4** | **40** | **47** | **53** | **4** |
| **4. Index finger length** | **64** | **72** | **79** | **5** | **60** | **67** | **74** | **4** |
| **5. Middle finger length** | **76** | **83** | **90** | **5** | **69** | **77** | **84** | **5** |
| **6. Ring finger length** | **65** | **72** | **80** | **4** | **59** | **66** | **73** | **4** |
| **7. Little finger length** | **48** | **55** | **63** | **4** | **43** | **50** | **57** | **4** |
| **8. Thumb breadth (IPJ)^{a}** | **20** | **23** | **26** | **2** | **17** | **19** | **21** | **2** |
| **9. Thumb thickness (IPJ)** | **19** | **22** | **24** | **2** | **15** | **18** | **20** | **2** |
| **10. Index finger breadth (PIPJ)^{b}** | **19** | **21** | **23** | **1** | **16** | **18** | **20** | **1** |
| **11. Index finger thickness (PIPJ)** | **17** | **19** | **21** | **1** | **14** | **16** | **18** | **1** |
| **12. Hand breadth (metacarpal)** | **78** | **87** | **95** | **5** | **69** | **76** | **83** | **4** |
| **13. Hand breadth (across thumb)** | **97** | **105** | **114** | **5** | **84** | **92** | **99** | **5** |
| **14. Hand breadth (minimum)^{c}** | **71** | **81** | **91** | **6** | **63** | **71** | **79** | **5** |
| **15. Hand thickness (metacarpal)** | **27** | **33** | **38** | **3** | **24** | **28** | **33** | **3** |
| **16. Hand thickness (including thumb)** | **44** | **51** | **58** | **4** | **40** | **45** | **50** | **3** |
| **17. Maximum grip diameter^{d}** | **45** | **52** | **59** | **4** | **43** | **48** | **53** | **3** |
| **18. Maximum spread** | **178** | **206** | **234** | **17** | **165** | **190** | **215** | **15** |
| **19. Maximum functional spread^{e}** | **122** | | **142 162** | **12** | **109** | **127** | **145** | **11** |
| **20. Minimum square access^{f}** | **56** | **66** | **76** | **6** | **50** | **58** | **67** | **5** |

From Table 1 above, an average width of a hand of men is (78+87+95)/3 = 82.3 with SD = 5. An average width of a hand of women is (69+79+83)/3=79.6 with SD=4. An average width of an average human hand is (82.3+79.6)/2 = 80.95 with SD=4.5. Therefore, the above given example where the required pullback length is 80 mm and the catheter handle is 133 mm long, meets the requirements necessary for the pullback portion 46 to be securely contained inside the ergonomic catheter handle 124. That is, in above example, the length of handle body 123 is L1 = 133 mm, pullback length is 80 mm smaller than the length L2 of the pullback portion 46.

Similarly, with respect to the handle diameter, an average of the maximum grip diameter for men is (45+52+59)/3=52mm with SD=4, and an average of the maximum grip diameter for women is (43+48+53)/3=48 mm with SD=3. Therefore, an average maximum grip for an average human hand is (52+48)/2 = 50 mm with an average SD - (4+3)/2 = 3.5.

The ergonomic catheter handle 24 disclosed herein provides several advantages and novel features including, but not limited to, improved ease of handling of the PIU. In particular, during PIU draping / undraping and handoffs, the novel catheter handle 24 provides complementary heavy duty protection of the pullback portion 46 of the probe's imaging core 48 to prevent the imaging core 48 from bending.

According to at least on embodiment, a medical apparatus comprises: a stationary console in direct communication with a patient interface unit and an elongated flexible catheter probe having a handle for attachment to and detachment from the patient interface unit. When attached to the patient interface unit, the handle is adapted to enable single-hand manipulations of the patient interface unit and catheter as a unit-body (unibody). In some embodiments, the elongated flexible catheter probe includes an imaging core adapted for a linear pullback motion along a lengthwise direction of the catheter probe. In some embodiments, the flexible catheter probe comprises a proximal rigid pullback portion contained within the handle. In some embodiments, the handle includes a reinforced portion at the proximal end thereof adapted to support and protect the proximal rigid pullback portion of the catheter probe. The handle also includes a strain relief sleeve at the distal end thereof adapted to prevent kinking and suppress transmission of bending or vibration forces from the proximal rigid pullback portion to the flexible catheter probe.

In operation, the system computer 107 controls the PIU 10 and imaging core 48 according to the desired imaging procedure. For example, for OCT imaging, the system computer 107 may control the PIU 10 and imaging core 48 according to a desired pullback length, pullback rate, and image frame rate where each image frame corresponds to one cross-sectional image (typically one full revolution of the imaging core).

The ergonomic handle disclosed herein can be used as part of a medical system comprising: an intravascular imaging probe having a tubular shaft, an elongated handle permanently attached to a proximal portion the tubular shaft, and an imaging core arranged in a lumen that spans through the elongated handle and through the tubular shaft; a patient interface unit comprising a housing with an opening configured to receive the elongated handle, and a pullback unit attached to the housing and configured to engage with the imaging core to move the imaging core in a lengthwise direction through the lumen of the tubular shaft; and a system console in direct communication with the patient interface unit, wherein the system console includes a computer system configured to send command signals to control and operate the pullback unit, wherein the elongated handle includes a passage configured to receive therein the proximal portion of the tubular shaft, and wherein, when the elongated handle is connected to the pullback unit, the console sends commands to the pullback unit to engage with the imaging core and move the imaging core a predetermined pullback length in a linear direction through the lumen of the tubular shaft and into the housing of the patient interface unit.

In the medical system according to the above embodiment, the elongated handle is ergonomically sized and shaped for grasping by a hand of a user, wherein a length of the elongated handle is equal to or greater than the predetermined pullback length which is a distance that the pullback unit moves the imaging core.

In the medical system according to the above embodiments, the length of the elongated handle is in a range of 1 to 2 times (or more preferably 1 to 1.5 times) the predetermined pullback length which the pullback unit moves the imaging core. In the medical system according to the above embodiment, in a case where the predetermined pullback length is about 80 mm, the length of the elongated handle is at least 133 mm. In the medical system according to the above embodiment, the elongated handle has a triangular cross-section or a circular cross-section configured to fit into a circle having a diameter of at least 25 mm. In the medical system according to the above embodiment, at least part of the elongated handle includes knurl-like features configured to prevent slippage from the hand of the user. In the medical system according to the above embodiment, the handle body includes a reinforce portion, such that the handle is adapted for support, handling, and manipulations of the entire patient interface unit.

In the medical system according to the above embodiment, to attach the handle to the patient interface unit, the user grasps the handle body with a single hand, inserts the coupling mechanism into a receiving port of the patient interface unit, and rotates the handle body single handedly. After attachment of the handle to the patient interface unit, the reinforced portion of the handle body abuts against a housing of the patient interface unit, and the coupling mechanism and the reinforce portion make the handle body robust enough to support the weight of the entire patient interface unit during handling and manipulations of the catheter handle and patient interface unit by the user.

In one embodiment, e.g., as shown in FIG. 2, the PIU 10 may have a weight in a range of 3 to 6 pounds (lbs), for example, approximately 5.6 pounds. In another embodiment, the PIU 10 may a weight in a range of 3 to 4 lbs, for example, approximately 3.8 lbs. In one embodiment, as described above, the catheter handle 24 engages with PIU 10 by inserting the coupling mechanism 21 into the receiving port 16. To maintain a rigid connection between the handle 24 and PIU 10, a length L4 of the handle receptacle 43 is substantially equal to the length L3 of the coupling mechanism 21. Given the above examples, and assuming an average PIU weight of approximately 3.5 pounds, the handle-to-PIU connection can withstand a moment of 6.2 Nm (newton-meter) in any direction perpendicular to the axis Ax. The foregoing data is compliant with current Standard ANSI AAMI HE75:2009/(R)2018 Human factors engineering - Design of medical devices.

In describing example embodiments illustrated in the drawings, specific terminology is employed for the sake of clarity. However, the disclosure of this patent specification is not intended to be limited to the specific terminology so selected, and it is to be understood that each specific element includes all technical equivalents that operate in a similar manner. Therefore, the scope of the following claims is to be accorded the broadest reasonable interpretation so as to encompass all such modifications and equivalent structures and functions.

Any patent, pre-grant patent publication, or other publication, in whole or in part, that is said to be incorporated by reference herein is incorporated only to the extent that the incorporated materials do not conflict with standard definitions or terms, or with statements and descriptions set forth in the present disclosure. As such, and to the extent necessary, the disclosure as explicitly set forth herein supersedes any conflicting material incorporated by reference.

A handle for maneuvering a catheter, comprises: a handle body having an outer surface and an inner surface, the inner surface defining a tubular passage extending along a longitudinal axis from a proximal end to a distal end of the handle body. The handle includes a coupling mechanism at the proximal end and a strain relief sleeve at the distal end of the handle body. The strain relief sleeve is adapted to irremovably secure a flexible catheter sheath to the handle; and the coupling mechanism is adapted to detachably connect the handle to a patient interface unit (PIU). The handle body is ergonomically sized and shaped for grasping by a single hand of a user, and is configured to linearly engage to and disengage from the PIU via the coupling mechanism. When the handle is attached to the PIU, an imaging core moves linearly inside the tubular passage without bending.

## Claims

1. A handle for maneuvering a catheter, the handle comprising:
a handle body having an outer surface and an inner surface, the inner surface defining a tubular passage extending along a longitudinal axis from a proximal end to a distal end of the handle body;
wherein the handle includes a coupling mechanism at the proximal end, a strain relief sleeve at the distal end, and a reinforced portion between the proximal and distal ends of the handle body,
wherein the strain relief sleeve at the distal end of the handle body is adapted to irremovably secure a flexible catheter sheath to the handle,
wherein the coupling mechanism at the proximal end of the handle body is adapted to detachably connect the handle to a patient interface unit, and
wherein the reinforced portion between the proximal and distal ends of the handle body is configured to increase a flexural rigidity at the proximal end of the handle body.

2. The handle according to claim 1,
wherein the handle body is ergonomically sized and shaped for grasping by a hand of a user so that the user can connect or disconnect the handle to or from the patient interface unit single handedly.

3. The handle according to claim 1,
wherein a length of the handle body is approximately equal to an average width of an average human hand.

4. The handle according to claim 1,
wherein the outer surface of the handle body defines a triangular cross-section or a circular cross-section configured to fit into a circle having a diameter smaller than or equal to a maximum grip diameter of an average human hand.

5. The handle according to claim 1,
wherein the outer surface of the handle body defines a triangular cross-section or a circular cross-section configured to fit into a circle having a diameter equal to or greater than 25 millimeters and equal to or smaller than a maximum grip diameter of an average human hand.

6. The handle according to claim 1,
wherein the catheter sheath includes a working channel spanning from a proximal end to a distal end of the catheter sheath, and
wherein the tubular passage of the handle body and the working channel of the catheter sheath are linearly aligned with each other and configured to pass theretrough an imaging core.

7. The handle according to claim 6,
wherein the patient interface unit includes a moving platform configured to linearly pull the imaging core a predetermined pullback length into the patient interface unit while the catheter sheath and the handle body remain stationary, and
wherein a length of the handle body is equal to or greater than the predetermined pullback length.

8. The handle according to claim 7,
wherein the length of the handle body is in a range of 1 to 1.5 times the predetermined pullback length.

9. The hand according to claim 1,
wherein the handle body and the catheter sheath are sterile components configured for single use, and
wherein the patient interface unit is an unsterile electronic component configured for multiuse.

10. The handle according to claim 1,
wherein the reinforced portion is a portion of the handle body at the proximal end of thereof that tapers from a first diameter to a second diameter smaller than the first diameter in a direction from the proximal end towards the distal end,
wherein, to attach the handle to the patient interface unit, the user grasps the handle body with a single hand, inserts the coupling mechanism into a receiving port of the patient interface unit, and rotates the handle body single handedly.

11. The hand according to claim 10,
wherein, after attachment of the handle to the patient interface unit, the reinforced portion of the handle body abuts against a housing of the patient interface unit, and
wherein the coupling mechanism and the reinforce portion make the handle body robust enough to support the weight of the entire patient interface unit during handling and manipulations of the catheter handle and patient interface unit by the user.

12. A medical apparatus comprising:
a catheter probe spanning in a longitudinal direction along a longitudinal axis from a proximal end to a distal end;
a handle body having an outer surface and an inner surface, the inner surface defining a tubular passage extending from a proximal end to a distal end of the handle body;
an imaging core arranged in a working channel that extends through the tubular passage of the handle body and through the catheter probe along the longitudinal axis; and
a patient interface unit having a connection port and a pullback unit,
wherein the handle includes a coupling mechanism at the proximal end, a strain relief sleeve at the distal end, and a reinforced portion between the proximal and distal ends of the handle body,
wherein the strain relief sleeve at the distal end of the handle body is adapted to irremovably secure a proximal end of the catheter probe to the handle body,
wherein the coupling mechanism at the proximal end of the handle body is adapted to detachably connect the handle body to the patient interface unit, and
wherein the reinforced portion between the proximal and distal ends of the handle body is configured to increase a flexural rigidity at the proximal end of the handle body.

13. The apparatus according to claim 12,
wherein the handle body is configured to be grasped by a user's hand so that the user can connect the catheter probe to the patient interface unit through the connection port, and
wherein the pullback unit is configured to simultaneously rotate and linearly move the imaging core while the catheter probe remains stationary.

14. The apparatus according to claim 12,
wherein the pullback unit includes a hollow shaft motor configured to rotate the imaging core, and a movable platform configured to linearly pull the imaging core to the inside of the patient interface unit a predetermined pullback length.

15. The apparatus according to claim 12,
wherein the handle body is ergonomically sized and shaped for grasping by a hand of a user, and
wherein a length of the handle body is equal to the greater of: (a) an average width of an average human hand or (b) the predetermined pullback length.

16. The apparatus according to claim 12,
wherein a length of the handle body is in a range of 1 to 1.5 times the predetermined pullback length.

17. The apparatus according to claim 12,
wherein the handle body has a substantially circular cross-section or a triangular cross-section with rounded edges configured to fit into a circle having a diameter equal to or greater than 25 mm and equal to or smaller than a maximum grip diameter of an average human hand.

18. The apparatus according to claim 17,
wherein at least part of the handle body having the substantially circular cross-section or the triangular cross-section with rounded edges includes knurl-like features configured to prevent slippage of a hand of a user.

19. The apparatus according to claim 12,
wherein the catheter probe and handle body are sterile and configured for single use, and
wherein the patient interface unit is unsterile and configured for multiuse.

20. The apparatus according to claim 12,
wherein the imaging core includes a rigid pullback portion arranged in the tubular passage of the handle body, a first connector attached to a proximal end the imaging core,
wherein the pullback unit comprises a hollow shaft motor configured to rotate an optical fiber connected to a second connector, and
wherein the second connector is configured to engage with the first connector of the imaging core.

21. The apparatus according to claim 20,
wherein the rigid pullback portion includes a hypotube having a length equal to the length of the handle body, and
wherein the pullback unit is configured to pull the hypotube and the imaging core into the patient interface unit by controlling the moving platform to move the hypotube linearly in a direction from the distal end towards the proximal end, while the hollow shaft motor rotates the imaging core.

22. The apparatus according to claim 20,
wherein the coupling mechanism is hollow tube attached to or integrally built into the proximal end of the handle body,
wherein the first connector attached to the proximal end the imaging core is provided inside the hollow tube, and
wherein, when the pullback unit pulls the hypotube and the imaging core into the patient interface unit, the second connector pulls the first connector out of the hollow tube.
